# EUROPÄISCHE PATENTSCHRIFT

(11) **EP 2 925 372 B1**
(45) Veröffentlichungstag und Bekanntmachung des Hinweises auf die Patenterteilung: **11.01.2017**
(21) Anmeldenummer: 13799265.7
(22) Anmeldetag: 27.11.2013
(51) Int. Cl.: A61L 2/08, B65B 55/02, B67C 7/00, H01J 33/02, H01J 37/30

(54) **VORRICHTUNG UND VERFAHREN ZUM STERILISIEREN VON BEHÄLTNISSEN MIT RÖNTGENSTRAHLUNGSÜBERWACHUNG**
DEVICE AND METHOD FOR STERILIZING CONTAINERS, COMPRISING AN X-RAY RADIATION MONITORING SYSTEM
DISPOSITIF ET PROCÉDÉ DE STÉRILISATION DE RÉCIPIENTS AVEC SURVEILLANCE DU RAYONNEMENT X

(30) Priorität: 27.11.2012 DE 102012111494
(43) Veröffentlichungstag der Anmeldung: 07.10.2015
(73) Patentinhaber: Krones AG, 93073 Neutraubling (DE)
(72) Erfinder: KRÜGER, Jochen, 93073 Neutraubling (DE)
(74) Vertreter: Bittner, Bernhard
(86) Internationale Anmeldenummer: PCT/EP2013/074857
(87) Internationale Veröffentlichungsnummer: WO 2014/083054

(56) Entgegenhaltungen:
- EP-A1- 2 316 495
- EP-A1- 2 462 953
- WO-A1-2006/108453
- WO-A1-2010/121775
- WO-A1-2013/092735

## Beschreibung

Die vorliegende Erfindung bezieht sich auf eine Vorrichtung und ein Verfahren zum Sterilisieren von Behältnissen und insbesondere von Kunststoffbehältnissen. Aus dem Stand der Technik ist es bekannt, dass Behältnisse vor ihrer Abfüllung sterilisiert werden, insbesondere bei der Abfüllung mit bestimmten sensiblen Getränken. Zum Zwecke dieser Sterilisierung sind unterschiedliche Vorgehensweisen bekannt, wie etwa die Beaufschlagung mit einem Sterilisationsmittel wie Peressigsäure oder Wasserstoffperoxid. In jüngerer Zeit ist man jedoch bestrebt, den Einsatz derartiger Chemikalien zurückzudrängen.

EP 2 462 953 A1 beschreibt eine Vorrichtung zum Sterilisieren von Behältnissen mittels Elektronenstrahlung, die einen Sensor für diese Strahlung aufweist.

WO2010121775 offenbart eine Vorrichtung zur Überwachung der Intensität eines Elektronenstrahles, wobei ein Detektor zur messtechnischen Erfassung einer direkt oder indirekt vom Elektronenstrahl erzeugten Elektronenstrahlung oder elektromagnetischen Strahlung ausgebildet ist und dass der Detektor mit einer Auswertungseinrichtung zur Erkennung von Änderungen der Intensität der vom Elektronenstrahl erzeugten Elektronenstrahlung oder elektromagnetischen Strahlung verbunden ist.

WO2006108453, Stand der Technik nach Art. 54(3) EPÜ, offenbart eine Vorrichtung zum Sterilisieren von Behältnissen mittels Elektronenstrahlung oder Röntgenstrahlung, die einen Sensor für diese Strahlungsarten aufweist. Allgemein ist jedoch die Sterilisation von Behältnissen, wobei im Folgenden unter Behältnissen sowohl fertig ausgebildete Behältnisse, wie Kunststoffbehältnisse oder Glasflaschen verstanden werden, als auch etwa Kunststoffvorformlinge, welche zu Flaschen expandiert werden sollen, neben dem eigentlichen Füllvorgang der zentrale Prozessschritt in einer insbesondere aseptischen Abfüllanlage. Bei der Entkeimung der Behältnisse mittels Strahlen, insbesondere Elektronenstrahlen, ist zum einen die Sicherheit der Behandlung, d.h. die Sterilisationswirkung auf jeden einzelnen Behälter, wichtig. Ein weiteres, wichtiges Kriterium ist jedoch auch die Sicherheit der Bediener und des Personals an der Anlage, insbesondere, da bei der Beaufschlagung mit beispielsweise Elektronenstrahlen an sich unterwünschte Nebeneffekte, wie Röntgenstrahlung, auftreten.

Bei der Bestrahlung der Behältnisse mit hochenergetischen Elektronen entsteht, wie erwähnt, als unerwünschter Nebeneffekt auch Röntgenstrahlung. Um die Personen und auch elektronische Komponenten in der Umgebung der Maschine vor dieser Strahlung zu schützen, werden üblicherweise Abschirmungen angebracht, die aus einem Material mit ausreichender Wandstärke bestehen. Für Elektronenstrahlen mit einer Energie im Bereich von 150 keV werden zur Abschirmung der Röntgenstrahlung Bleiwände mit einer Dicke von 5 - 12 mm benötigt (dies hängt auch von der Stromstärke ab). Alternativ kann auch ein anderes Material, wie zum Beispiel Stahl, verwendet werden, jedoch ist die Abschirmwirkung von Stahl deutlich geringer, so dass die Wandstärke entsprechend dicker auszulegen ist.

Jedenfalls ist die komplette Anlage derart abgeschirmt, dass bei den definierten Parametern der Strahlung und einem bestimmungsgemäßen, d.h. normalen Betrieb der Anlage, an keiner Stelle außerhalb der Anlage eine Reststrahlung von mehr als 1 µSv/h austreten kann. Es kann jedoch auch ein Störfall auftreten, wenn zum Beispiel einer oder mehrere der Strahler defekt sind oder falsch angesteuert werden, so dass eine erhöhte Strahlung produziert wird. Um diesen bestimmungsgemäßen oder "normalen" Betrieb sicherzustellen, werden Detektoren bzw. Sensoreinrichtungen eingesetzt. Aus dem internen Stand der Technik ist eine Überwachung dieser Strahlung mittels Röntgendetektoren bekannt.

Diese Sensoreinrichtungen bzw. Strahlendetektoren müssen über viele Betriebsstunden hinweg zuverlässig funktionieren, da sie letztlich die Strahlensicherheit der Maschine überwachen und notfalls eine Notausschaltung einleiten. Im normalen Betrieb tritt der Fall einer überhöhten Strahlung in der Regel nicht auf, so dass die Sensoren über Tage, Wochen oder Monate hinweg keinen Störfall feststellen und keinen Notstopp einleiten. Daher ist es notwendig, die Sensoreinrichtungen in regelmäßigen Zeitabständen auf Ihre Funktion zu überprüfen. Zu diesem Zweck sind aus dem Stand der Technik mehrere Möglichkeiten bekannt.

Bei einer Vorgehensweise kann die Empfindlichkeit des Sensors elektronisch erhöht werden, bzw. dessen Messsignal zusätzlich verstärkt werden. Die Auswertung des Signals ergibt ein vermeintlich zu hohes Strahlungsniveau, was zum Abschalten der Emitter führen muss. Eine weitere Möglichkeit der Überprüfung besteht darin, den Sensor einem überhöhten Strahlungsniveau auszusetzen, so dass er gegebenenfalls, seiner Aufgabe entsprechend, die Strahlenquellen abschalten muss. Dies könnte im einfachen Fall erreicht werden, indem das normale Strahlungsniveau im Inneren der Maschine erhöht wird. Allerdings sollte dies aus Aspekten der Strahlensicherheit vermieden werden. Beide genannten Möglichkeiten haben jedoch den Nachteil, dass das System manipuliert wird und dass sicherheitsrelevante Komponenten der Maschine verändert werden. Der Sensor, dessen Elektronik und auch die Auswerteeinheit sind Bestandteil einer Sicherheitstechnik.

Der Erfindung liegt daher die Aufgabe zugrunde, eine derartige Überwachung der Anlagen zu ermöglichen, ohne hierzu die im Stand der Technik erforderlichen Manipulationen an sicherheitsrelevanten Bauteilen vorzunehmen.

Diese Aufgaben werden erfindungsgemäß durch die Gegenstände der unabhängigen Ansprüche erreicht.

Vorteilhafte Ausführungsformen und Weiterbildungen sind Gegenstand der Unteransprüche.

Eine erfindungsgemäße Vorrichtung zum Sterilisieren von Behältnissen weist eine Transporteinrichtung auf, welche die Behältnisse entlang eines vorgegebenen Transportpfades transportiert. Weiterhin weist die Vorrichtung eine Strahlungseinrichtung auf, welche die Behältnisse und/oder Bestandteile der Vorrichtung zum Zwecke deren Sterilisation mit einer Strahlung wenigstens abschnittsweise beaufschlagt. Weiterhin ist auch eine Sensoreinrichtung vorgesehen, welche eine infolge der von der Strahlungseinrichtung ausgehenden Strahlung auftretende (insbesondere weitere) Strahlung detektiert.

Erfindungsgemäß weist die Vorrichtung ein Abschirmelement auf, welches die zu der Sensoreinrichtung gelangende Strahlung in einem Arbeitsbetrieb der Vorrichtung abschwächt und eine Bewegungseinrichtung zum Bewegen dieses Abschirmelements gegenüber der Sensoreinrichtung bzw. zum Erzeugen einer Relativbewegung zwischen der Sensoreinrichtung und dem Abschirmelement.

Es wird daher erfindungsgemäß vorgeschlagen, das Strahlungsniveau, welchem die Sensoreinrichtung im Normalbetrieb ausgesetzt ist, zu erhöhen, ohne das Mess- oder Strahlersystem zu manipulieren. Die Möglichkeit besteht darin, die auf die Sensoreinrichtung gelangende Strahlung im Normalbetrieb durch ein Abschirmelement zu dämpfen. Dadurch erfährt die Sensoreinrichtung ein permanent schwächeres Strahlungssignal, als es tatsächlich in der Maschine vorhanden ist.

Um die Sensoreinrichtung zu testen, wird das Abschirmelement bevorzugt von der Sensoreinrichtung weggezogen und die Sensoreinrichtung detektiert nun ein wesentlich stärkeres Röntgensignal, obwohl das Strahlungsniveau in der Maschine unverändert (und auch sicher) ist und auch die Sensoreinrichtung selbst nicht verändert wurde. In der Folge müsste die Sensoreinrichtung nun einen Alarm auslösen und die Abschaltung der Strahlungsquellen einleiten.

Es wäre jedoch auch möglich, nicht das Abschirmelement zu bewegen sondern die Sensoreinrichtung, etwa die Sensoreinrichtung aus einem von dem Abschirmelement geworfenen Schatten heraus zu bewegen, was beispielsweise durch eine lineare Bewegung und/oder eine Schwenkbewegung der Sensoreinrichtung erfolgen kann.

Bevorzugt handelt es sich bei der von der Sensoreinrichtung zu erfassenden Strahlung um Röntgenstrahlung. Röntgenstrahlung wird beim Durchgang durch Materie deutlich geschwächt. Die Strahlung tritt mit der durchdrungenen Materie in Wechselwirkung und die Energie der Photonen wird aufgrund von Photoeffekt und Compton-Streuung im Material absorbiert. Die Intensität der durchdringenden Strahlung nimmt nach dem Gesetz von Lambert und Beer mit der im Material zurückgelegten Strecke exponentiell ab (I= I₀ x e^{-kx}). Dabei ist der Massenabschwächungskoeffizient k abhängig von dem durchstrahlten Material und der Wellenlänge der Strahlung. Im Stand der Technik sind zahlreiche Tabellenwerke für derartige Abschwächungskoeffizienten bekannt. Es wäre jedoch auch möglich, dass zum Sterilisieren der Behältnisse unmittelbar Röntgenstrahlung eingesetzt wird. Unter einem Sterilisieren wird im Rahmen dieser Offenbarung ein Prozess verstanden, der dazu dient, die Zahl der vermehrungsfähigen Mikroorganismen zumindest auf ein bestimmtes Maß zu reduzieren.

So kann beispielsweise errechnet werden, dass ein Blech aus Eisen mit einer Stärke von 2 mm die Intensität einer Röntgenstrahlung, die durch Elektronen mit einer Beschleunigungsspannung von 90 kV erzeugt wurden, um etwa 50% abschwächt.

Genauer wird beispielsweise die Röntgenstrahlung, welche bei mit einer Beschleunigungsspannung von 90 kV beschleunigten Elektronen entsteht mit einem 1 mm dicken Eisenblech um 32% abgeschwächt, mit einem 2 mm starken Eisenblech um 53%, mit einem 3 mm starken Eisenblech um 68%, mit einem 4 mm starken Eisenblech um 78% und mit einem 5 mm starken Eisenblech um 85%. Bei einer Beschleunigungsspannung von 125 kV beträgt die Abschwächung bei einem 1 mm starken Eisenblech 18%, bei einem 2 mm starken Eisenblech 33%, bei einem 3 mm starken Eisenblech 45%, bei einem 4 mm starken Eisenblech 55% und bei einem 5 mm starken Eisenblech 63%. Diese Werte lassen sich auch experimentell bestätigen.

Bevorzugt handelt es sich um eine Strahlungseinrichtung, welche stationär angeordnet ist, und an der sich die Behältnisse vorbeibewegen. In diesem Falle dient die Strahlungseinrichtung insbesondere zur Bestrahlung der Außenoberflächen der Behältnisse. Es wäre jedoch auch möglich, dass die Strahlungseinrichtung zur Innenbehandlung der Behältnisse dient und beispielsweise einen stangenartigen Körper oder Strahlfinger aufweist, der in das Innere der Behältnisse eingeführt wird. In diesem Falle werden diese Strahlungseinrichtungen bzw. Strahlfinger bevorzugt mit den Behältnissen mit bewegt.

Bei einer weiteren vorteilhaften Ausführungsform weist die Vorrichtung sowohl Strahlungseinrichtungen zur Außenbehandlung der Behältnisse als auch Strahlungseinrichtungen zur Innenbehandlung der Behältnisse auf. Die Strahlungseinrichtung kann jedoch auch dazu dienen, um Elemente der Vorrichtung zu sterilisieren, etwa Greifklammern, welche die Behältnisse während deren Sterilisation halten oder auch andere Bereiche der Sterilisationseinrichtung wie etwa Gehäusewandungen oder dergleichen. Auch können mehrere derartige Sterilisationseinrichtungen zu unterschiedlichen Zwecken eingesetzt sein.

Bevorzugt weist die Vorrichtung einen Reinraum auf, innerhalb dessen die Behältnisse während ihrer Sterilisation transportiert werden. Dabei ist vorteilhaft dieser Reinraum mittels wenigstens einer Wandung gegenüber einer (insbesondere unsterilen) Umgebung abgegrenzt. Weiterhin können auch Dichtungseinrichtungen vorgesehen sein, welche den Reinraum, bzw. bezüglich einander bewegliche Wandungen des Reinraums, abdichten. Dabei kommt beispielsweise ein sogenanntes Wasserschloss infrage, welches einen umlaufenden mit einer Flüssigkeit gefüllten Kanal aufweist, in dem ein (insbesondere drehbewegliches) Schwert, welches mit der jeweiligen anderen Wandung in Verbindung steht, eintaucht.

Bei einer weiteren vorteilhaften Ausführungsform weist die Transporteinrichtung zum Transportieren der Behältnisse ein Trägerrad auf, an dem eine Vielzahl von Halteeinrichtungen zum Halten der Behältnisse angeordnet ist. Bei dieser Ausgestaltung werden die Behältnisse bevorzugt während ihrer Sterilisation entlang eines kreisförmigen Transportpfads geführt.

Bei einer weiteren vorteilhaften Ausführungsform handelt es sich bei den zu sterilisierenden Behältnissen um Kunststoffvorformlinge oder um Kunststoffflaschen.

Bei einer weiteren vorteilhaften Ausführungsform ist die Bewegungseinrichtung derart gestaltet, dass sie das Abschirmelement wenigstens teilweise aus einem zu der Sensoreinrichtung gelangenden Strahlengang der Strahlung entfernen kann. Mit anderen Worten kann die Sensoreinrichtung bzw. ein Detektorelement in einem Normalbetrieb der Anlage von dem Abschirmelement verdeckt sein und nur zu Testzwecken das Abschirmelement entfernt werden.

Dabei ist es bevorzugt auch möglich, dass das Abschirmelement während eines laufenden Betriebs entfernt wird, oder allgemein während des laufenden Betriebs die besagte Relativbewegung zwischen dem Abschirmelement und der Sensoreinrichtung vorgenommen wird. Auf diese Weise ist es insbesondere möglich, die Strahlungseinrichtung und/oder die Sensoreinrichtung im laufenden Betrieb zu überprüfen.

Vorteilhaft ist die Bewegungseinrichtung derart gestaltet, dass das Abschirmelement innerhalb eines vorgegebenen Zeitraums aus dem Strahlengang entfernt werden kann, der kleiner ist als 200ms, bevorzugt kleiner als 100ms, bevorzugt kleiner als 50ms und besonders bevorzugt kleiner als 30ms. Vorteilhaft ist eine derartige Messung innerhalb des Arbeitsbetriebs, also insbesondere einschließlich des Entfernens des Abschirmelements, der eigentlichen Messung und des anschließenden wieder Zuführen des Abschirmelements in einer zeitlichen Dauer möglich, die kürzer ist als 200ms, bevorzugt kürzer als 100ms, bevorzugt kürzer als 50ms, bevorzugt kürzer als 30ms und welche bevorzugt in einem Bereich zwischen 20ms und 30ms liegt.

Dabei ist weiterhin bevorzugt eine Steuerungseinrichtung vorgesehen, welche bei der Überprüfung der Sensoreinrichtung auch eine Position der zu sterilisierenden Behältnisse berücksichtigt. Bevorzugt ist die Steuerungseinrichtung derart beschaffen, dass die jeweiligen Überprüfungen stets an einem gleichen Ort der Behältnisse vorgenommen werden, also stets dann, wenn sich die Behältnisse an einer vorbestimmten und gleichbleibenden Position bezüglich der Sensoreinrichtung befinden. Auf diese Weise kann eine Vergleichbarkeit mehrerer Überprüfungsmessungen gewährleistet werden.

Vorteilhaft weist die Vorrichtung auch eine Speichereinrichtung auf, in der Messwerte derartiger Überprüfungsmessungen ablegbar sind. Auf diese Weise ist es möglich, Langzeitmessungen durchzuführen bzw. ein Langzeitprotokoll aufzunehmen.

Es wäre jedoch auch möglich, dass die Überprüfungsmessungen an unterschiedlichen Behältnispositionen durchgeführt werden. Bei dieser Vorgehensweise wird jedoch die jeweilige Position der zu sterilisierenden Behältnisse mit erfasst. Bevorzugt weist die Vorrichtung daher auch eine Erfassungseinrichtung auf, welche eine Relativposition der Behältnisse während der Überprüfung der Sensoreinrichtung erfasst.

Bei einer weiteren vorteilhaften Ausführungsform ist die Strahlungseinrichtung eine Elektronenstrahlungseinrichtung, welche Elektronenstrahlen ausgibt. Genauer gesagt handelt es sich hierbei um beschleunigte Elektronen. Vorteilhaft weist daher die Strahlungseinrichtung eine Beschleunigungseinrichtung zum Beschleunigen der Elektronen auf. Vorteilhaft weist die Strahlungseinrichtung auch ein Austrittsfenster auf, über welches die beschleunigten Elektronen aus einem Gehäuse, innerhalb dessen sie beschleunigt werden, austreten können. Vorteilhaft ist dabei dieses Austrittsfenster aus Titan hergestellt. Bei einer weiteren vorteilhaften Ausführungsform weist das Austrittsfenster eine Dicke auf, die zwischen 4 µm und 30 µm, bevorzugt zwischen 6 µm und 20 µm liegt. Vorteilhaft weist die Vorrichtung auch eine Kühleinrichtung zum Kühlen dieses Austrittsfensters auf.

Bei einer weiteren vorteilhaften Ausführungsform werden die Elektronen mit einer Beschleunigungsspannung beschleunigt, welche zwischen 50 kV und 300 kV, bevorzugt zwischen 60 kV und 250 kV, bevorzugt zwischen 70 kV und 200 kV, bevorzugt zwischen 70 kV und 150 kV und besonders bevorzugt zwischen 80 kV und 150 kV liegt. Es wäre jedoch auch eine Sterilisation mittels anderen Ladungsträgern denkbar, etwa mittels Protonen, alpha-Teilchen und dergleichen.

Bei einer weiteren vorteilhaften Ausführungsform ist die Sensoreinrichtung zur Erfassung von Röntgenstrahlen geeignet und bestimmt. Vorteilhaft unterscheiden sich daher die Strahlungen, mit welchen die Behältnisse beaufschlagt werden und die von der Sensoreinrichtung zu erfassende Strahlung. Vorteilhaft ist jedoch die von der Sensoreinrichtung zu erfassende Strahlung ein Resultat derjenigen Strahlung, mit der die Behältnisse sterilisiert werden. Insbesondere handelt es sich dabei um Bremsstrahlung bzw. Röntgenstrahlung.

Bei einer weiteren vorteilhaften Ausführungsform schwächt das Abschirmelement die auf die Sensoreinrichtung treffende Strahlung um wenigstens 10%, bevorzugt um wenigstens 20%, bevorzugt um wenigstens 30% und besonders bevorzugt um wenigstens 50% ab.

Das Strahlniveau im Inneren der Maschine ist im normalen Betrieb so hoch, dass auch eine Abschwächung um 30 bis 50% noch immer sehr hohe Messwerte liefert, die sehr gut ausgewertet werden können. Andererseits ist jedoch die Sensoreinrichtung selbst im Normalbetrieb nicht den hohen Belastungen ausgesetzt wie im Falle des (zeitlich sehr kurzen) Testbetriebs.

Vorteilhaft weist die Sensoreinrichtung einen Szintillator mit einem besonders bevorzugt nachgeschalteten Photomultiplier sowie besonders bevorzugt auch ein Zählrohr auf. Alternativ kann die Sensoreinrichtung auch ein Zählrohr oder einen Halbleiterdetektor oder einen anderen Detektor enthalten, der geeignet ist, Röntgenstrahlung quantitativ und mit geeigneter Genauigkeit zu erfassen.

Um die Elektronik der Sensoreinrichtung vor permanenter und zu starker Strahlenbelastung zu schützen, ist die Sensoreinrichtung vorteilhaft in einem strahlungsdichten Gehäuse untergebracht, welches Wandungen mit mehreren Millimetern Wandstärke aufweist. Bevorzugt weist daher die Sensoreinrichtung ein diese umgebendes Gehäuse auf. Dieses Gehäuse kann beispielsweise als Bleimantel ausgeführt sein. Vorteilhaft ist in diesem Gehäuse eine Öffnung vorgesehen, die im Normalbetrieb von dem Abschirmelement zumindest teilweise und besonders bevorzugt vollständig verschlossen ist. Bei einer weiteren vorteilhaften Ausführungsform sind auch Vorspannmittel vorgesehen, welche das Abschirmelement in eine geschlossene Stellung drängen. Dies bedeutet, dass das Abschirmelement in der Regel die auf die Sensoreinrichtung treffende Strahlung abschwächt.

Bei der Bewegungseinrichtung kann es sich um beliebige Bewegungseinrichtungen handeln, wie beispielsweise elektrische Antriebe, hydraulische Antriebe, pneumatische Antriebe und dergleichen. Es wäre auch als Bewegungseinrichtung eine manuelle Bewegungseinrichtung denkbar, so dass der Benutzer beispielsweise manuell das Abschirmelement aus dem auf die Sensoreinrichtung treffenden Strahlengang verfahren kann. So kann es sich bei der Bewegungseinrichtung beispielsweise um einen kleinen Schieber handeln, der seitlich in eine Bohrung vor dem Sensor integriert ist und durch Druckluft temporär aus der Bohrung entfernt wird. Dabei kann eine Druckfeder diesen Schieber nach dem Test wieder zurück in seine Position vor einer Blendeneinrichtung, durch welche die Strahlung zu der Sensoreinrichtung gelangt, verschieben.

Ist der Test erfolgreich (was sich beispielsweise dadurch äußert, dass die Anlage ordnungsgemäß abgeschaltet wurde), kann das Dämpfungsblech bzw. das Abschirmelement wieder in dem Strahlengang positioniert werden und die Maschine kann wieder in den Produktionsbetrieb gebracht werden. Wird trotz eingeschalteten Emittern in diesem Test kein automatischer Alarm ausgelöst, ist dies ein Zeichen, dass die Sensoreinrichtung nicht bestimmungsgemäß funktioniert oder dass die Blendeneinrichtung (z.B. aus mechanischen Gründen) nicht aus dem Strahlengang entfernt wurde. In diesem Fall sollte die Anlage ebenfalls abgeschaltet werden und nach dem Fehler gesucht werden.

Die vorliegende Erfindung ist weiterhin auf ein Verfahren zum Sterilisieren von Behältnissen gerichtet, wobei die Behältnisse entlang eines vorgegebenen Transportpfades transportiert werden und wobei die Behältnisse oder Bestandteile der Vorrichtung durch Beaufschlagung mit einer Strahlung wenigstens abschnittsweise sterilisiert werden. Dabei wird von einer Sensoreinrichtung infolge dieser Strahlung auftretende weitere Strahlung detektiert.

Erfindungsgemäß schwächt ein Abschirmelement die auf die Sensoreinrichtung gelangende Strahlung ab und dieses Abschirmelement wird wenigstens zeitweise (bezüglich der Sensoreinrichtung) derart bewegt, dass die auf die Sensoreinrichtung gelangende Strahlung weniger geschwächt wird. Es wird daher auch verfahrensseitig vorgeschlagen, dass - insbesondere in einem normalen Arbeitsbetrieb, bzw. Sterilisationsbetrieb - die auf die Sensoreinrichtung gelangende Strahlung permanent abgeschwächt wird. Vorteilhaft wird - insbesondere im Rahmen eines Testbetriebs - das Abschirmelement derart bewegt, dass die auf die Sensoreinrichtung gelangende Strahlung nicht oder weniger durch dieses Abschirmelement geschwächt wird.

Mit anderen Worten wird zum Zwecke einer Überprüfung, insbesondere der Sensoreinrichtung, das Abschirmelement derart bewegt, dass die auf Sensoreinrichtung gelangende Strahlung weniger geschwächt wird. Auch verfahrensseitig wäre es möglich, dass die Relativbewegung zwischen der Sensoreinrichtung und dem Abschirmelement durch eine Bewegung der Sensoreinrichtung erreicht wird.

Vorteilhaft erfasst die Sensoreinrichtung Röntgenstrahlung. Vorteilhaft gibt die Sensoreinrichtung ein Signal aus, welches für die auf die Sensoreinrichtung einwirkende Röntgenstrahlung charakteristisch ist.

Weitere Vorteile und Ausführungsformen ergeben sich aus den beigefügten Zeichnungen:
Darin zeigen:
   - Fig. 1: Eine schematische Darstellung einer erfindungsgemäßen Vorrichtung in einem ersten Betriebszustand;
   - Fig. 2: die Vorrichtung aus Fig. 1 in einem zweiten Betriebszustand;
   - Fig. 3: eine schematische Darstellung einer Sensoreinrichtung in einem ersten Betriebszustand;
   - Fig. 4: die Sensoreinrichtung in einem zweiten Betriebszustand;
   - Fig. 5: eine erfindungsgemäße Sensoreinrichtung in einem zweiten Betriebszustand;
   - Fig. 6: eine Darstellung einer Ausführungsform eines Abschirmelements in Form eines Shutterrads; und
   - Fig. 7: eine Darstellung von Messwerten bei Verwendung eines Shutterrads.

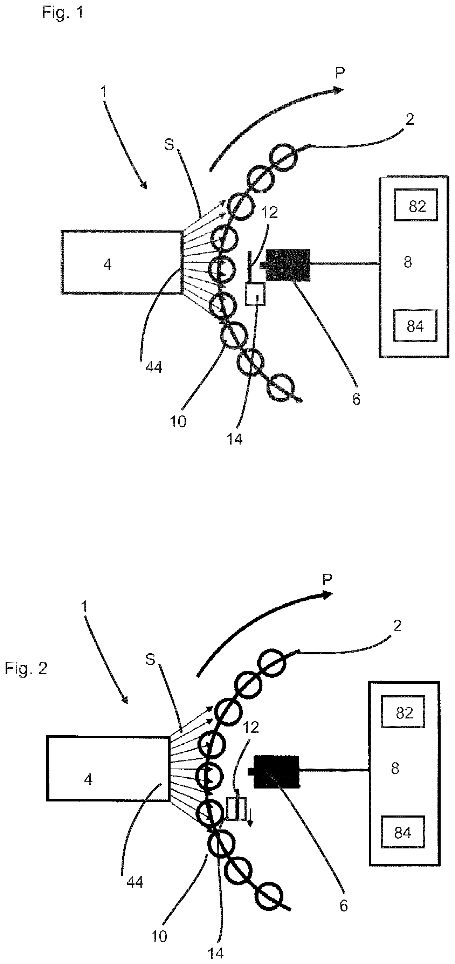

Fig. 1 zeigt eine erfindungsgemäße Vorrichtung 1 zum Sterilisieren von Behältnissen 10 in einem ersten Betriebszustand. Dabei werden die Behältnisse 10, hier Kunststoffflaschen oder Kunststoffvorformlinge, mittels einer Transporteinrichtung 2, die hier nur teilweise dargestellt ist und beispielsweise als drehbarer Träger ausgebildet ist, entlang eines vorgegebenen Transportpfades P transportiert. Während dieses Transports werden die Behältnisse 10 von einer Strahlungseinrichtung 4 und insbesondere einer Elektronenstrahlungseinrichtung 4 mit Strahlung S und insbesondere mit Elektronen beaufschlagt. Dabei treten diese Elektronen aus der Vorrichtung 4 über ein Austrittsfenster 44 aus. Durch diese Beaufschlagung werden insbesondere die Außenwandungen der Kunststoffbehältnisse sterilisiert.

Das Bezugszeichen S kennzeichnet hier die auf die Behältnisse auftreffende Strahlung. Infolge dieser Bestrahlung der Behältnisse tritt auch (als Nebeneffekt) Röntgenstrahlung auf, welche von einer Sensoreinrichtung 6 erfasst wird. Das Bezugszeichen 8 bezieht sich auf eine Auswerteeinrichtung, welche die auf die Sensoreinrichtung 6 auftreffende Strahlung auswertet, beispielsweise im Hinblick auf deren Intensität. Diese Auswerteeinrichtung 8 weist dabei eine Anzeigeeinrichtung 82 auf, welche dem Benutzer ein Signal, bzw. eine Angabe ausgibt, welche für die detektierte Röntgenstrahlung charakteristisch ist. Das Bezugszeichen 84 kennzeichnet eine Vergleichseinrichtung, welche die von der Sensoreinrichtung 6 erfasste Strahlung mit vorgegebenen Referenzwerten vergleicht. Das Bezugszeichen 12 kennzeichnet ein Abschirmelement, welches in einem Arbeitsbetrieb hier vor der Sensoreinrichtung 6, bzw. einen Sensorelement dieser Sensoreinrichtung 6 angeordnet ist und so die von der Sensoreinrichtung 6 erfasste Strahlung abschwächt.

Das Bezugszeichen 14 kennzeichnet eine Bewegungseinrichtung, welche zum Bewegen des Abschirmelements 12 dient. Es wäre dabei ebenfalls möglich, nicht das Abschirmelement 12 zu bewegen, sondern die Sensoreinrichtung 6 gewissermaßen aus dem Schatten den Abschirmelements heraus zubewegen. In jedem Falle wird erreicht, dass das Abschirmelement 12 nicht mehr in dem auf die Sensoreinrichtung 6 auftretenden Strahlengang angeordnet ist.

Fig. 2 zeigt die Vorrichtung aus Fig. 1, wobei nunmehr das Abschirmelement 12 beiseitegeschoben wurde und somit der Strahlengang ungehindert auf die Sensoreinrichtung 6 treffen kann. Dabei sind im Wesentlichen beliebige Bewegungen dieses Abschirmelements 12 denkbar, um dieses zumindest teilweise aus dem Strahlengang zu entfernen.

Vorteilhaft ist dieses Abschirmelement ein Stahlblech mit einer vorgegebenen Dicke, wodurch auch eine entsprechend vorgegebene Abschirmung bzw. Abschwächung der Röntgenstrahlung erreicht wird. Es wäre jedoch auch möglich, dass dieses Abschirmelement eine variable, bzw. sich ändernde Dicke aufweist, um so auch unterschiedliche Abschirmwirkungen zu erzielen. Dies wäre relevant, falls nicht nur qualitativ eine Aussage über die Funktion der Sensoreinrichtung getroffen werden soll, sondern auch quantitativ dessen Funktionsweise überprüft werden soll. Auch durch derartige quantitative Messungen können sich Rückschlüsse, beispielsweise über den Alterungszustand der Sensoreinrichtung, ergeben.

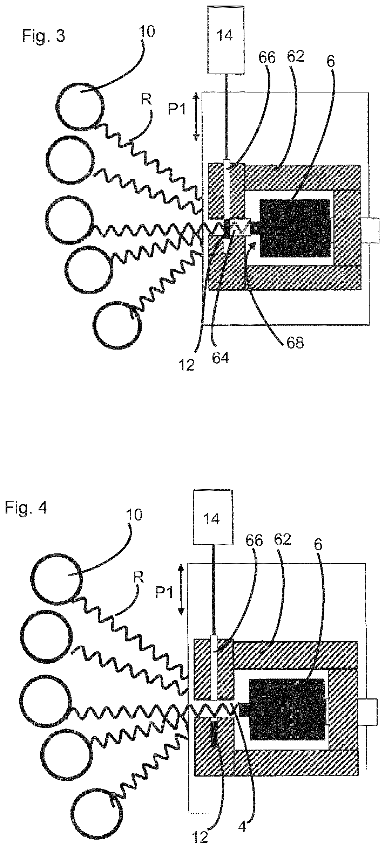

Fig. 3 zeigt eine Anordnung einer Sensoreinrichtung 6. Diese Sensoreinrichtung weist dabei ein insbesondere die empfindlichen elektronischen Bauteile umgebendes Gehäuse 62 auf. In diesem Gehäuse ist eine Öffnung bzw. Blende 64 ausgebildet, durch welche Röntgenstrahlung zu dem eigentlichen Sensorelement gelangen kann. Das Bezugszeichen R bezieht sich auf die von der Sensoreinrichtung 6 zu erfassende (Röntgen)strahlung. Das Bezugszeichen 68 kennzeichnet damit grob schematisch ein Sensorelement der Sensoreinrichtung 6, auf welches die Röntgenstrahlung auftrifft.

Das Bezugszeichen 66 kennzeichnet hier eine Nut, bzw. eine Ausnehmung, innerhalb der das Abschirmelement gleiten kann, bzw. beweglich ist. Bei der in Fig. 3 gezeigten Situation ist das Abschirmelement derart positioniert, dass es die Öffnung 64 verdeckt. Eine Verschiebung des Abschirmelements ist beispielsweise über Druckluft möglich, wobei bei ausgeschalteter Druckluft das Abschirmelement in der in Fig. 3 gezeigten Position ist.

Fig. 4 zeigt die Anordnung aus Fig. 3, wobei hier jedoch das Abschirmelement 12 so verschoben ist, dass Strahlung auch durch die Öffnung 64 zu der Sensoreinrichtung 6 gelangen kann, ohne durch das Abschirmelement 12 geschwächt zu werden. In diesem Falle kann beispielsweise Druckluft über die Ausnehmung 66 zugeführt werden, um das Abschirmelement 12 zu verschieben.

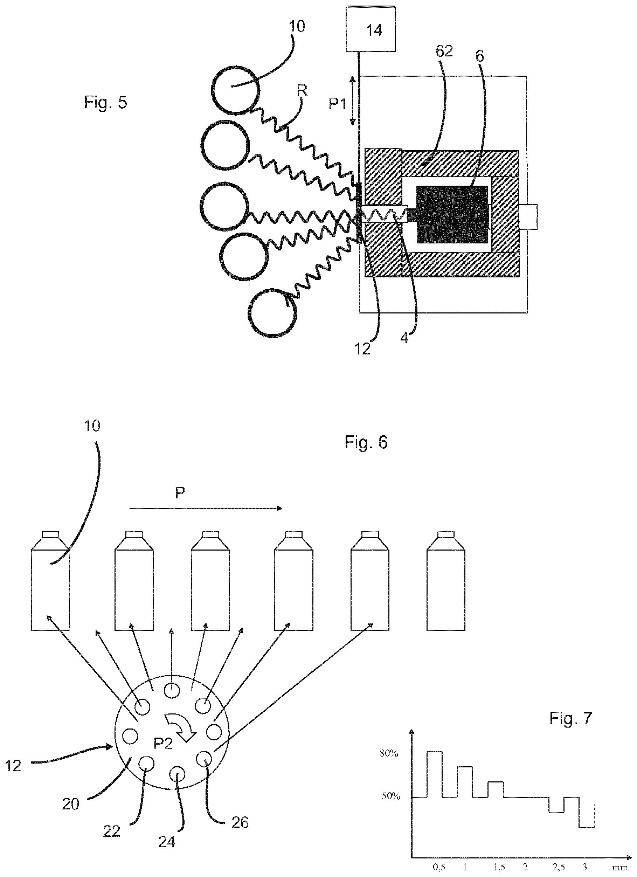

Fig. 5 zeigt eine weitere Ausführungsform einer erfindungsgemäßen Sensoreinrichtung. Hier ist das Abschirmelement 12 an einem Hebel angeordnet, der dessen Verschiebung in Richtung des Doppelpfeils P1 erlaubt. Auch auf diese Weise kann erreicht werden, dass die Strahlung entweder ungeschwächt zu der Sensoreinrichtung 6 gelangen kann oder aber bei der entsprechend anderen Stellung des Abschirmelements 12 durch dieses geschwächt wird.

Fig. 6 zeigt eine weitere Ausgestaltung der erfindungsgemäßen Vorrichtung, wobei das Abschirmelement hier als drehbares Element bzw. als Shutterrad ausgeführt ist. Dieses Abschirmelement 12 weist dabei einen Träger 20 auf, der eine bestimmte Dicke aufweist bzw. eine bestimmte Abschirmung der Strahlung bewirkt. In diesem Träger ist eine Vielzahl von Fenstern 22, 24, 26 eingesetzt, die jeweils unterschiedliche Dicken aufweisen und damit unterschiedliche Abschirmungen bewirken. Die Behältnisse 10 werden hier in Richtung des Pfeils P bewegt. Das Abschirmelement ist, wie durch den Pfeil P2 gezeigt, drehbar.

Fig. 7 zeigt einen beispielhaften Intensitätsverlauf bei unterschiedlichen Stellungen des in Fig. 6 gezeigten Abschirmelements. Man erkennt, dass der Träger 20 hier eine Abschirmung bzw. Dämpfung um 50% bewirkt. Die einzelnen Fenster 22, 24, 26 bewirken hiervon abweichende Dämpfungen, hier von 80%, 70%, 60%, 40% und 30%. Durch den Einsatz eines derartigen Abschirmelements 12 in Form eines Shutterrads ist es möglich, mehrere unterschiedliche Abschirmeffekte einzustellen bzw. zu simulieren.

### Bezugszeichenliste

- 1: Vorrichtung
- 2: Transporteinrichtung
- 4: Strahlungseinrichtung
- 6: Sensoreinrichtung
- 8: Auswerteeinrichtung
- 10: Behältnisse
- 12: Abschirmelement
- 14: Bewegungseinrichtung
- 20: Träger
- 22, 24, 26: Fenster
- 44: Austrittsfenster
- 62: Gehäuse
- 64: Öffnung
- 66: Nut/Ausnehmung
- 68: Sensorelement
- 82: Anzeigeeinrichtung
- 84: Vergleichseinrichtung
- P: Transportpfad/Doppelpfeil
- S: Strahlung
- R: zu erfassende Strahlung
- P1: Bewegungsrichtung des Abschirmelements 12
- P2: Drehrichtung des Abschirmelements

## Patentansprüche

1. Vorrichtung (1) zum Sterilisieren von Behältnissen (10) mit einer Transporteinrichtung (2), welche die Behältnisse (10) entlang eines vorgegebenen Transportpfads (P) transportiert, mit einer Strahlungseinrichtung (4), welche die Behältnisse (10) zum Zwecke deren Sterilisation wenigstens abschnittsweise mit einer Elektronenstrahlung (S) beaufschlagt und mit einer Sensoreinrichtung (6), welche eine in Folge der von der Strahlungseinrichtung (6) ausgegebenen Elektronenstrahlung (S) auftretende Röntgenstrahlung detektiert,
**dadurch gekennzeichnet, dass**
die Vorrichtung (1) ein Abschirmelement (12) aufweist, welches die zu der Sensoreinrichtung (6) gelangende Röntgenstrahlung in einem Arbeitsbetrieb der Vorrichtung (1) abschwächt, sowie eine Bewegungseinrichtung (14) zum Bewegen dieses Abschirmelements (12) gegenüber der Sensoreinrichtung (6), wobei die Sensoreinrichtung (6) in einem Normalbetrieb der Vorrichtung (1) von dem Abschirmelement (12) verdeckt ist und das Abschirmelement (12) nur zu Testzwecken der Sensoreinrichtung (6) bewegt wird.

2. Vorrichtung (1) nach Anspruch 1,
**dadurch gekennzeichnet, dass**
die Bewegungseinrichtung (14) derart gestaltet ist, dass sie das Abschirmelement (12) wenigstens teilweise aus einem zu der Sensoreinrichtung (6) gelangenden Strahlengang der Strahlung entfernen kann.

3. Vorrichtung (1) nach wenigstens einem der vorangegangenen Ansprüche,
**dadurch gekennzeichnet, dass**
die Strahlungseinrichtung (4) eine Elektronenstrahleinrichtung ist, welche Elektronenstrahlung ausgibt.

4. Vorrichtung (1) nach wenigstens einem der vorangegangenen Ansprüche,
**dadurch gekennzeichnet, dass**
die Sensoreinrichtung (6) zur Erfassung von Röntgenstrahlung geeignet und bestimmt ist.

5. Vorrichtung (1) nach wenigstens einem der vorangegangenen Ansprüche,
**dadurch gekennzeichnet, dass**
das Abschirmelement (12) die auf die Sensoreinrichtung treffende Strahlung um wenigstens 10%, bevorzugt um wenigstens 20%, bevorzugt um wenigstens 30%, bevorzugt um wenigstens 50% abschwächt.

6. Vorrichtung (1) nach wenigstens einem der vorangegangenen Ansprüche,
**dadurch gekennzeichnet, dass**
die Vorrichtung (1) ein die Sensoreinrichtung (6) umgebendes Gehäuse (62)aufweist.

7. Verfahren zum Sterilisieren von Behältnissen (10), wobei die Behältnisse (10) entlang eines vorgegebenen Transportpfades (P) transportiert werden und wobei die Behältnisse (10) oder Bestandteile einer Vorrichtung zum Sterilisieren von Behältnissen (10) durch Beaufschlagung mit einer Elektronenstrahlung wenigstens abschnittsweise sterilisiert werden, und wobei eine Sensoreinrichtung (6) infolge dieser Elektronenstrahlung auftretende Röntgenstrahlung detektiert,
**dadurch gekennzeichnet, dass**
ein Abschirmelement (12) die auf die Sensoreinrichtung (6) gelangende Röntgenstrahlung abschwächt und dieses Abschirmelement (12), zum Zwecke einer Überprüfung der Sensoreinrichtung (6), wenigstens zeitweise derart bezüglich der Sensoreinrichtung (6) mittels einer Bewegungseinrichtung (14), welche zum Bewegen des Abschirmelements (12) dient, bewegt wird, dass die auf die Sensoreinrichtung (6) gelangende Röntgenstrahlung weniger geschwächt wird.

8. Verfahren nach Anspruch 7,
**dadurch gekennzeichnet, dass**
die Sensoreinrichtung (6) Röntgenstrahlung erfasst.

9. Verfahren nach Anspruch 7,
**dadurch gekennzeichnet, dass**
die Strahlungseinrichtung (4) die Behältnisse oder Bestandteile der Vorrichtung mit Ladungsträgern und insbesondere mit Elektronen beaufschlagt.

## Claims

1. Apparatus (1) for sterilizing containers (10), comprising a transport device (2) which transports the containers (10) along a predefined transport path (P), a radiation device (4) which applies radiation (S) to at least some sections of the containers (10) for sterilization purposes, and a sensor device (6) which detects X-ray radiation occurring as a result of the electron radiation (S) emitted by the radiation device (6),
**characterized in that**
the apparatus (1) has a shielding element (12) which attenuates the X-ray radiation reaching the sensor device (6) in a working mode of the apparatus (1), and a movement device (14) for moving said shielding element (12) relative to the sensor device (6) wherein the sensor device (6) is concealed by the shielding element (12) in a normal operation mode of the apparatus (1) and the shielding element (12) is moved only for test purposes of the sensor device (6).

2. Apparatus (1) according to claim 1,
**characterized in that**
the movement device (14) is configured in such a way that it can remove the shielding element (12) at least partially from a beam path of the radiation reaching the sensor device (6).

3. Apparatus (1) according to at least one of the preceding claims,
**characterized in that**
the radiation device (4) is an electron beam device which emits electron radiation.

4. Apparatus (1) according to at least one of the preceding claims,
**characterized in that**
the sensor device (6) is suitable for and is intended for detecting X-ray radiation.

5. Apparatus (1) according to at least one of the preceding claims,
**characterized in that**
the shielding element (12) attenuates the radiation impinging on the sensor device by at least 10%, preferably by at least 20%, preferably by at least 30%, preferably by at least 50%.

6. Apparatus (1) according to at least one of the preceding claims,
**characterized in that**
the apparatus (1) has a housing (62) surrounding the sensor device (6).

7. Method for sterilizing containers (10), wherein the containers (10) are transported along a predefined transport path (P), and wherein the containers (10) or components of an apparatus for sterilizing containers (10) are sterilized at least in some sections by being exposed to electron radiation, and wherein a sensor device (6) detects X-ray radiation occurring as a result of said electron radiation,
**characterized in that**
a shielding element (12) attenuates the radiation reaching the sensor device (6) and this shielding element (12) is moved at least at times relative to the sensor device (6) for the purpose of checking the sensor device (6) by means of a movement device (14) which serves to move the shielding element (12) such that the radiation reaching the sensor device (6) is attenuated to a lesser degree.

8. Method according to claim 7,
**characterized in that**
the sensor device (6) detects X-ray radiation.

9. Method according to claim 7,
**characterized in that**
the radiation device (4) applies charge carriers and in particular electrons to the containers or to components of the apparatus.

## Revendications

1. Dispositif (1) de stérilisation de récipients (10) comprenant un système de transport (2), lequel transportant les récipients (10) le long d'un chemin de transport (P) prédéfini, un système d'émission de rayonnement (4), lequel appliquant un rayonnement électronique (S) au moins sur certaines parties des récipients (10) afin de les stériliser, et un système de détection (6), lequel détectant un rayonnement X consécutif au rayonnement électronique (S) émis par le système d'émission de rayonnement (6),
**caractérisé en ce que**
le dispositif (1) comprend un élément de blindage (12), lequel atténue le rayonnement X parvenant au système de détection (6) lors du fonctionnement du dispositif (1), ainsi qu'un système de déplacement (14) servant à déplacer cet élément de blindage (12) par rapport au système de détection (6), le système de détection (6) étant dissimulé par l'élément de blindage (12) lors d'un fonctionnement normal du dispositif (1) et l'élément de blindage (12) n'étant déplacé qu'à des fins de test du système de détection (6).

2. Dispositif (1) selon la revendication 1,
**caractérisé en ce que**
le système de déplacement (14) est réalisé de telle manière qu'il peut retirer l'élément de blindage (12) au moins en partie d'un faisceau du rayonnement parvenant au système de détection (6).

3. Dispositif (1) selon au moins l'une des revendications précédentes,
**caractérisé en ce que**
le système d'émission de rayonnement (4) est un système d'émission de rayonnement électronique, lequel émet un rayonnement électronique.

4. Dispositif (1) selon au moins l'une des revendications précédentes,
**caractérisé en ce que**
le système de détection (6) est adapté et destiné à détecter un rayonnement X.

5. Dispositif (1) selon au moins l'une des revendications précédentes,
**caractérisé en ce que**
l'élément de blindage (12) atténue le rayonnement incident sur le système de détection d'au moins 10 %, de préférence d'au moins 20 %, de préférence d'au moins 30 %, de préférence d'au moins 50 %.

6. Dispositif (1) selon au moins l'une des revendications précédentes,
**caractérisé en ce que**
le dispositif (1) comprend un boîtier (62) entourant le système de détection (6).

7. Procédé de stérilisation de récipients (10), les récipients (10) étant transportés le long d'un chemin de transport (P) prédéfini et les récipients (10) ou des composants d'un dispositif de stérilisation de récipients (10) étant stérilisés au moins sur certaines parties à la suite de l'application d'un rayonnement électronique, et un système de détection (6) détectant un rayonnement X consécutif à ce rayonnement électronique,
**caractérisé en ce que**
un élément de blindage (12) atténue le rayonnement X parvenant au système de détection (6) et cet élément de blindage (12), afin de contrôler le système de détection (6), est déplacé au moins par intermittence par rapport au système de détection (6) au moyen d'un système de déplacement (14), lequel sert à déplacer l'élément de blindage (12), de telle sorte que le rayonnement X parvenant au système de détection (6) est moins atténué.

8. Procédé selon la revendication 7,
**caractérisé en ce que**
le système de détection (6) détecte un rayonnement X.

9. Procédé selon la revendication 7,
**caractérisé en ce que**
le système d'émission de rayonnement (4) applique des porteurs de charge et en particulier des électrons aux récipients ou aux composants du dispositif.
